# EUROPEAN PATENT APPLICATION

(11) **EP 1 661 878 A1**
(43) Date of publication of application: **31.05.2006**
(21) Application number: 05023657.9
(22) Date of filing: 28.10.2005
(51) Int. Cl.: C07C 57/28, A61K 31/192, A61P 17/06, A61P 17/10

(54) **Adapalene polymorphic forms**

(30) Priority: 26.11.2004 IT MI20042290; 14.07.2005 IT MI20051350
(71) Applicant: Dipharma S.p.A., 33036 Mereto di Tomba (Udine) (IT); LUNDBECK PHARMACEUTICALS ITALY S.p.A., 35129 Padova (IT)
(72) Inventor: Ventimiglia, Gianpiero, 20092 Cinisello Balsamo (MI) (IT); Allegrini, Pietro, 20097 San Donato Milanese (MI) (IT)
(74) Representative: Minoja, Fabrizio

(57) **Abstract**

Novel adapalene crystalline and amorphous Forms, processes for the preparation thereof, their use for the purification of adapalene, pharmaceutical compositions containing said forms and the use thereof in therapy.

## Description

### FIELD OF THE INVENTION

The present invention relates to adapalene amorphous form and to an adapalene novel crystalline form, a process for the preparation thereof, the use of amorphous adapalene form and of the novel crystalline form in a process for the purification of adapalene, pharmaceutical compositions containing said novel forms and the use thereof in therapy.

### TECHNOLOGICAL BACKGROUND

Adapalene, namely 6-[3-(1-adamantyl)-4-methoxyphenyl]-2-naphthoic acid, having the following chemical formula: 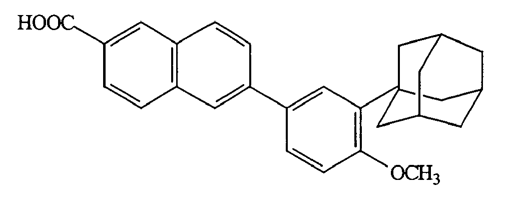 is disclosed in US 4,717,720 and used in dermatology, in particular for the treatment of *acne vulgaris* and psoriasis.

Different forms of biologically active compounds, such as polymorphs, are known to have different bioavailability, release time and solubility, which allow, for example, dose reduction or prolonged administration intervals. Moreover, the different physical properties that are often associated to different physical forms of medicaments can be advantageously exploited in the manufacture of pharmaceutical formulations.

There is therefore the need to provide novel polymorphic forms of biologically active compounds with advantageous properties.

### SUMMARY OF THE INVENTION

It has now been found that adapalene, in addition to the known crystalline form, in the following referred to as Form I, can also exist in an amorphous form and in a novel crystalline form, in the following referred to as crystalline Form α, which are stable at room temperature.

Therefore, the invention relates to adapalene amorphous Form and crystalline Form α, a process for the preparation thereof, a pharmaceutical composition containing said forms and the use thereof in therapy.

A further object of the present invention is a process for the purification of adapalene by use of said novel forms, to obtain adapalene of suitable quality to fulfil the regulatory requirements for therapeutical products.

### BRIEF DISCLOSURE OF THE FIGURES

The polymorphic forms of adapalene were characterized with the known XRPD (X-ray powder diffraction) technique. X-ray diffraction spectra (XRPD) were registered with an APD 2000 θ/θ automatic diffractometer for powders and liquids (Ital-Structures), under the following operative conditions: radiation CuKα (1.5418 Å), scanning 3-40° with a 0.03°C angular step for a time of 1 sec.
Figure 1. XRPD spectrum of adapalene amorphous Form;
Figure 2. XRPD spectrum of adapalene crystalline Form α.

According to present invention, when a particles sample is stated to have mean diameter, referred to as D[4,3], higher than X µm, this means that the mean volume of the particles constituting the sample is higher than the volume of a spherical particle with diameter X.

The particle sizes, i.e. mean diameter values D[4,3], were determined with the known technique of *laser light scattering* using a Malvern Mastersizer MS1 instrumentation under the following operative conditions:
- 300RF mm lens with laser beam length of 2.4 mm;
- 100 mg sample dispersed in 100 ml of isopar and 0.5 ml of a 2% solution of lecithin in isopar, with 3 min presonication and 2500 rpm stirring rate.

### DETAILED DISCLOSURE OF THE INVENTION

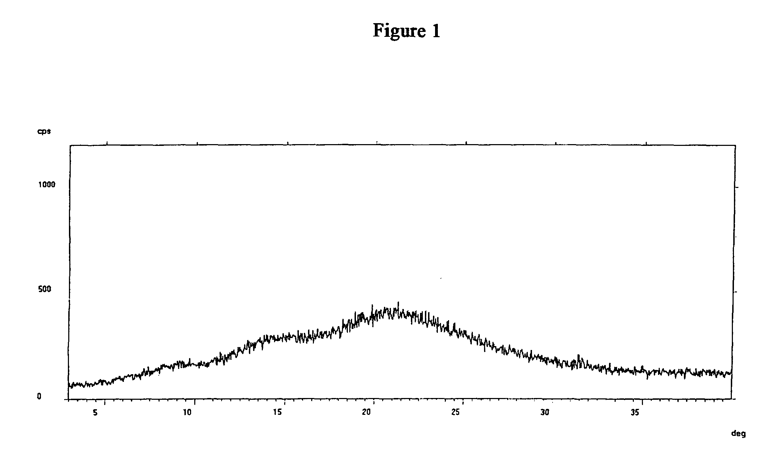

A first object of the present invention is adapalene amorphous Form, having the XRPD spectrum substantially as reported in Figure 1.

Adapalene amorphous Form can be prepared by a process which comprises the following steps:
- hot solubilization of crude adapalene in an organic polar aprotic solvent,
- precipitation of amorphous adapalene with an anti-solvent, and
- separation of the resulting amorphous adapalene.

According to the present invention, an organic polar aprotic solvent is, for example, a solvent selected from the group of dimethylsulfoxide, dimethylacetamide, dimethylformamide, tetrahydrofuran or mixtures thereof, in particular dimethylsulfoxide.

According to the present invention, an anti-solvent is for example water, an alkanol or mixtures thereof. Preferred examples of alkanols are methanol, ethanol, 1-propanol, 2-propanol. Preferred examples of water-alkanol mixtures are water-methanol and water-ethanol mixtures, more preferably water-methanol. The water to alcoholic solvent ratio can approx. range from 1:5 to 5:1, preferably from 2:1 1:2, in particular approx. 1:1.

Adapalene is solubilized in the organic solvent at a temperature ranging from approx. 40°C to the solvent reflux temperature, preferably at a temperature ranging from abou 55°C to the solvent reflux temperature, more preferably at a temperature having an approx. intermediate value. The adapalene concentration in the solution can approx. range from 5 to 50%, preferably from 15 to 30% w/v.

This solution is poured in an anti-solvent, kept at a temperature approx. ranging from 0 to 25°C, preferably from 4 to 8°C, and solid adapalene is separated in the amorphous form. This can be recovered with a process comprising the separation of the solid by filtration, followed by washing with the same anti-solvent as used above, final washing with a low-boiling organic solvent, such as ethyl ether, acetone or methanol, preferably acetone, and drying under vacuum.

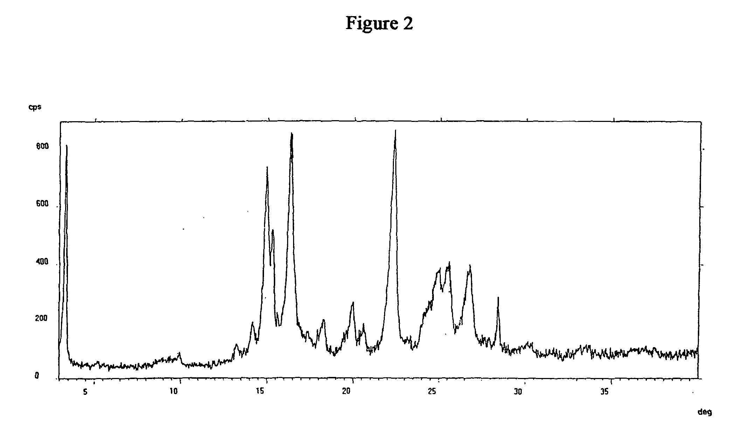

It has now been found that adapalene can exist in a further crystalline form, stable at room temperature, herein defined as Form α, which is a further object of the invention. Adapalene crystalline Form α has a XRPD spectrum substantially as reported in Figure 2, wherein the most intense diffraction peaks fall at 3.17; 14.75; 16.16; 22.13 and 25.22 ± 0.2° in 2θ.

Adapalene crystalline Form α can be advantageously obtained by a process comprising the following steps:
- heating a crude adapalene dispersion in a non-ester organic solvent or mixtures thereof with an ester solvent, until solubilization;
- cooling of the solution, and
- separation of the resulting solid.

Adapalene crystalline Form α can be obtained by dispersing crude adapalene in a non-ester organic solvent, for example selected from dimethylsulfoxide, dimethylacetamide, dimethylformamide and tetrahydrofuran, preferably dimethylsulfoxide and tetrahydrofuran, or in a mixture of one of these solvents with an ester solvent. According to the present invention, an ester solvent is for example a solvent selected from the group of ethyl acetate, isopropyl acetate, butyl acetate and isobutyl acetate, preferably ethyl acetate. A mixture of non-ester and ester solvents, for example tetrahydrofuran and ethyl acetate, is preferably a in ratio ranging from 50 to 90% v/v, more preferably approx. 70% v/v. Adapalene concentration in the starting solution can approx. range from 2 to 5%, preferably from 3 to 4%.

The dispersion is then heated to a temperature approx. ranging from 50°C to the reflux temperature, depending on the solvent, to solubilize crude adapalene. The resulting solution is cooled to room temperature or lower, thereby separating adapalene crystalline Form α. This is recovered, preferably by filtration, followed by washing with the same solvent mixture as used in the preparation of the dispersion, and subsequent drying, preferably under vacuum, at a temperature depending on the solvent mixture, typically ranging approx. from 30°C to the boiling temperature of the mixture, preferably approx. 50°C.

Crude adapalene used as the starting material for the preparation of both amorphous adapalene and adapalene crystalline Form α can be obtained for example as reported in US 4,717,720.

Adapalene amorphous Form and adapalene crystalline Form α, similarly to the known adapalene Form I, are useful in therapy in the treatment of dermatological pathologies, as reported above.

The invention also relates to a pharmaceutical composition comprising as the active ingredient adapalene amorphous Form and/or adapalene crystalline Form α, optionally in admixture with crystalline Form I, together with a diluent and/or carrier.

A preferred object of the invention is a pharmaceutical composition comprising as the active ingredient adapalene amorphous Form, together with a diluent and/or carrier.

A preferred object of the invention is a pharmaceutical composition comprising as the active ingredient adapalene crystalline Form α, together with a diluent and/or carrier and.

The choice of the adapalene amorphous Form, adapalene crystalline Form α and adapalene Form I ratios depends on the physical and biological properties thereof and will be suitably selected by those skilled in the art.

The pharmaceutical compositions of the invention can be formulated in a variety of pharmaceutical forms for the administration to humans or animals, according to known techniques. Such compositions can be for example in the form of creams, gel, suspensions, emulsions, solutions, capsules, tablets, sugar-coated pills or other forms known. When formulated e.g. as creams, gel or solutions, the amount of the active ingredient can approx. range from 0.02% to about 0.2%.

The processes reported above to obtain adapalene crystalline Form α and adapalene amorphous Form, allow to purify the final product from any impurities formed during the process for the synthesis of adapalene, deriving from side reactions and degradation of the product itself.

Therefore, a further object of the present invention is a process for the purification of adapalene, comprising the conversion of crude adapalene, as obtainable e.g. according to US 4,717,720, to adapalene crystalline Form α or adapalene amorphous Form and, if desired, the subsequent conversion of the novel resulting form, to the Form I, by crystallization under the conditions disclosed in US 4,717,720.

Said process affords adapalene amorphous Form, crystalline Form α, or crystalline Form I, with a purity above 99.9%, i.e. suitable to fulfil the regulatory requirements for products for the therapeutical use.

The particles of adapalene amorphous Form, crystalline Form α, crystalline Form I, as obtainable according to the invention, typically have mean D[4,3] diameter of 30 *µ*m or lower, preferably of 20 *µ*m or lower, more preferably of 15 *µ*m or lower.

If desired, after completion of the processes described above, the particles of adapalene amorphous Form, crystalline Form α or Form I can be subjected e.g. to fine grinding or micronisation, to obtain particles having lower mean D[4,3] diameter, typically of 5 *µ*m or lower, preferably of 2 *µ*m or lower.

Adapalene having said particle size distribution is particularly suitable for the formulation in pharmaceutical forms for the topical administration.

The following examples illustrate the invention.

### Example 1. Preparation of adapalene crystalline Form α

1 g of adapalene is dissolved in 30 ml of a tetrahydrofuran - ethyl acetate mixture (7:3 v/v) under reflux temperature (68°C). The resulting clear solution is cooled to about 15°C, in approx. 30 minutes, then filtered. The product is dried under vacuum at 50°C, to afford 0.900 g of adapalene crystalline Form α having HPLC purity higher than 99.5%, XRPD spectrum with characteristic peaks substantially as reported in Figure 2 and particles having mean D[4,3] diameter of approx. 15 *µ*m.

### Example 2. Preparation of adapalene crystalline Form α

1 g of adapalene is dissolved at about 90°C in 25 ml of dimethylsulfoxide. The resulting solution is cooled to 20°C, to afford 0.750 g of a solid. The XRPD spectrum of the product shows characteristic peaks substantially as reported in Figure 2, with particles having mean D[4,3] diameter of approx. 15 µm and HPLC purity higher than 99.5%.

### Example 3. Preparation of adapalene amorphous Form

1 g of adapalene is dissolved at about 90°C in 5 ml of dimethylsulfoxide. The hot solution is then poured into 20 ml of a water-methanol mixture (1:1 v/v), at a temperature of approx. 5°C. A gummy solid immediately forms, which is recovered by filtration, washed first with the water-methanol mixture, then with acetone, and finally dried under vacuum at 50°C. The resulting solid has XRPD spectrum substantially as reported in Figure 1, with particles having mean D[4,3] diameter of approx. 15 *µ*m and HPLC purity higher than 99.5%.

## Claims

1. Adapalene, 6-[3-(1-adamantyl)-4-methoxyphenyl]-2-naphthoic acid, in the amorphous Form.

2. A pharmaceutical composition comprising as the active ingredient adapalene amorphous Form, together with a diluent and/or carrier.

3. Adapalene crystalline Form α having XRPD spectrum wherein the most intense diffraction peaks fall at 3.17; 14.75; 16.16; 22.13 and 25.22 ± 0.2° in 2θ.

4. Adapalene crystalline Form α, according to claim 3, having a XRPD spectrum substantially as reported in Figure 2.

5. A pharmaceutical composition comprising as the active ingredient adapalene crystalline Form α, as defined in claim 3, together with a diluent and/or carrier.

6. Pharmaceutical composition comprising as the active ingredient adapalene amorphous Form and/or adapalene crystalline Form α, as defined in claim 3, optionally in admixture with adapalene crystalline Form I, together with a diluent and/or carrier.

7. A process for the preparation of adapalene amorphous Form comprising:
- hot solubilization of crude adapalene in an organic polar aprotic solvent,
- precipitation of amorphous adapalene with an anti-solvent, and
- separation of the resulting amorphous adapalene.

8. A process for the preparation of adapalene crystalline Form α, as defined in claim 3, comprising:
- heating of a crude adapalene dispersion in a non-ester organic solvent or mixtures thereof with an ester solvent, until solubilization;
- cooling of the solution, and
- separation of the resulting solid.

9. A process for the purification of adapalene comprising the conversion of crude adapalene to adapalene crystalline Form α, as defined in claim 3, or to adapalene amorphous Form and, if desired, the subsequent conversion of the resulting novel form to crystalline Form I.

10. Adapalene amorphous Form, crystalline Form α, as defined in claim 3, or crystalline Form I, having purity degree higher than 99.9%.

11. Adapalene amorphous Form, crystalline Form α, as defined in claim 3, or crystalline Form I in the form of particles typically having mean D[4,3] diameter of 30 *µ*m or lower.
